# EUROPEAN PATENT APPLICATION

(11) **EP 4 502 147 A1**
(43) Date of publication of application: **05.02.2025**
(21) Application number: 23382801.1
(22) Date of filing: 31.07.2023
(51) Int. Cl.: C12N 5/0786

(54) **METHOD FOR THE POLARIZATION OF CELLS TO A NEW M2 PHENOTYPE AND USES OF SAID M2-POLARIZED CELLS**

(71) Applicant: M2Rlab SL, 28006 Madrid (ES); Consejo Superior De Investigaciones Científicas, 28006 Madrid (ES)
(72) Inventor: HOTTER CORRIPIO, Georgina, 28006 Madrid (ES); JÁTIVA JIMÉNEZ, Soraya, 28006 Madrid (ES); TORRICO PONCE, Selene, 28006 Madrid (ES); GINESTA BUCH, Francesc Xavier, 28006 Madrid (ES); MUÑOZ HERRERO, María Ángeles, 28006 Madrid (ES); GARCÍA DE LA RIVA MAESTRE, Pablo, 28006 Madrid (ES)
(74) Representative: Hoffmann Eitle

(57) **Abstract**

The present invention provides a method for generating cells polarized to a M2 phenotype, comprising culturing mononuclear phagocyte system cells in a suitable culture medium comprising lactic acid or a lactate derivative, and subjecting the cells to a period of hypoxia followed by a period of re-oxygenation. The present invention also provides compositions comprising the cells obtained with the method defined herein and uses thereof.

## Description

### TECHNICAL FIELD

The present invention relates to the field of cell therapies for tissue repair. Particularly, the present invention relates to a method for the polarization of cells, particularly monocytes or macrophages, to a M2 phenotype, which are useful for tissue regeneration. Pharmaceutical compositions comprising said cells and their use in cell therapy in the regeneration of damaged, injured or impaired tissue are also included herein.

### BACKGROUND ART

A proper tissue repair and regeneration after an acute injury is key for a total restoration of the damaged tissue. Tissue regeneration is a complex process managed by different cellular types, and that follow three different overlapped phases: inflammation, tissue formation and remodeling. The activity of the different cell types involved in the process must be finely and timely balanced in order not only to promote the adequate resolution of the inflammatory phase, but also an efficient cell proliferation and differentiation in order to restore the destroyed tissue, and a proper remodeling in order to reduce the formation of fibrotic tissue.

Mononuclear cells, and more specifically macrophages and monocytes, are involved in all the phases of the regenerative process. In this sense, inflammatory macrophages are mainly present in the inflammatory process, removing the dead cells and tissue, and releasing factors that recruit other inflammatory cells and fibroblast to the injured area. Due their plasticity, macrophages con change their phenotype to an anti-inflammatory releasing factors that promote cell proliferation and differentiation.

Several subdivision of anti-inflammatory macrophages have been described, depending on the factors that promote their polarization, and the cytokine profile expressed. In this sense the most common M2 macrophages are M2A, M2B and M2C macrophages, which have in common an overexpression of key cytokines such as IL-10 but differ in the expression of other inflammatory cytokines like IL 6, IL 12, TNF-a and TGB.

The use of macrophages has raised a great attention in the last years, due to their key role in the regenerative process, and several are the approaches focused on the use of these cells. In this sense, several modified macrophages have been developed in order to enhance the expression of several cytokines and proteins that seems to be critical in the regenerative process, like (a) anti-inflammatory cytokines (b) enzymes that degrade Extracellular matrix (ECM) and (c) factors that promote cell regeneration.

Taking into account the exceptional plasticity of these cells and the different factors involved in the polarization process, an infinite amount of different phenotypes can be expected only changing the conditions in which this process is produced. The key approach should be focused not only in one specific factor, but on a specific phenotype that express and suppress the major number of factors that promote or reduce the regenerative potential of these cells.

In particular, methods for obtaining macrophages polarized to a phenotype that promotes wound healing and, in general, tissue regeneration, are required. Specifically, methods for the artificial polarization of macrophages into an M2 phenotype would be of special interest, as M2 macrophages present a phenotype that is important in the promotion of wound healing and tissue remodeling as well as in the resolution of inflammation. The development of these methods would therefore enable the artificial manipulation of macrophage polarization to obtain macrophages phenotypes which enhance normal physiological processes, such as wound repair.

### BRIEF DESCRIPTION OF DRAWINGS

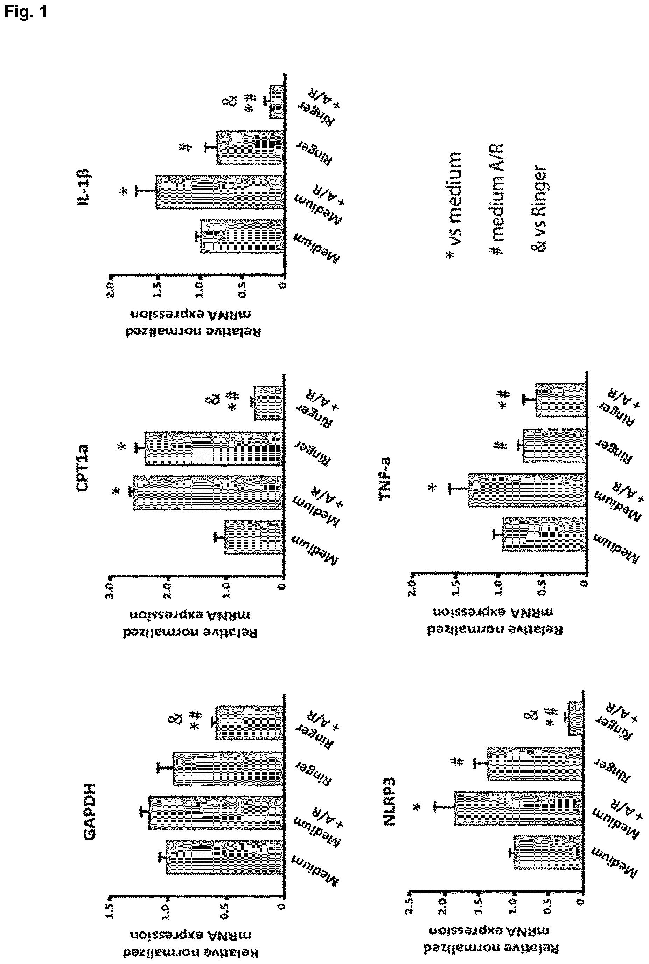

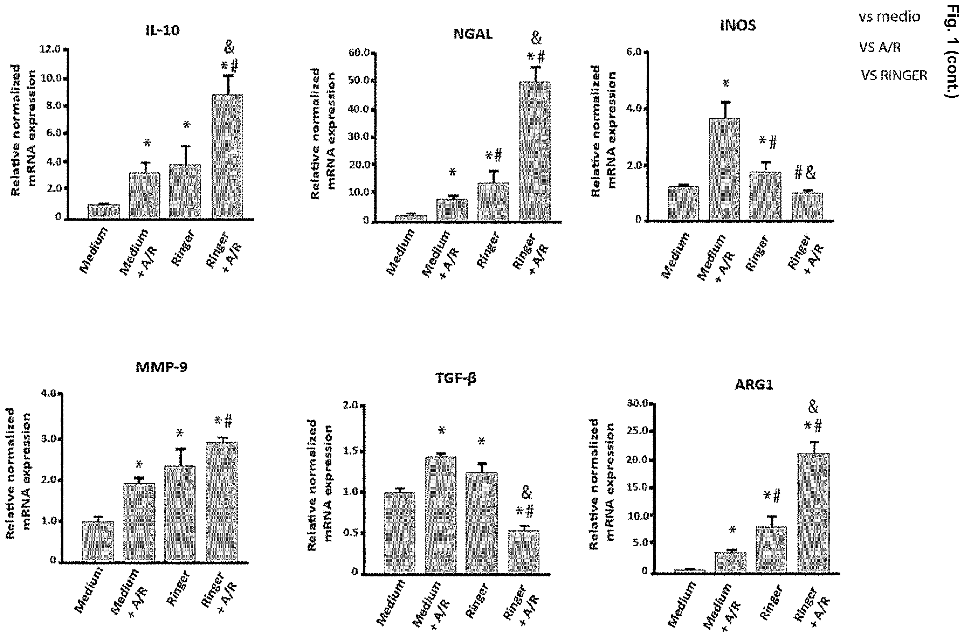

**Fig. 1****:** Gene expression profile of human monocytes (THP-1 cell line) in different conditions. Assessed groups: medium, medium+ Anoxia reoxygenation (AR), ringer and ringer+ Anoxia reoxygenation. mRNA levels of GAPDH, CPT1-α, IL-1β, NLRP3, TNF-α, TGF-β, iNOS, MMP-9, Arginase1, IL-10, NGAL normalized to actine and expressed as fold change. Data were presented as mean ± SEM; *P < 0.05 vs medium; # P < 0.05 vs ringer, & P <0.05 vs medium AR.

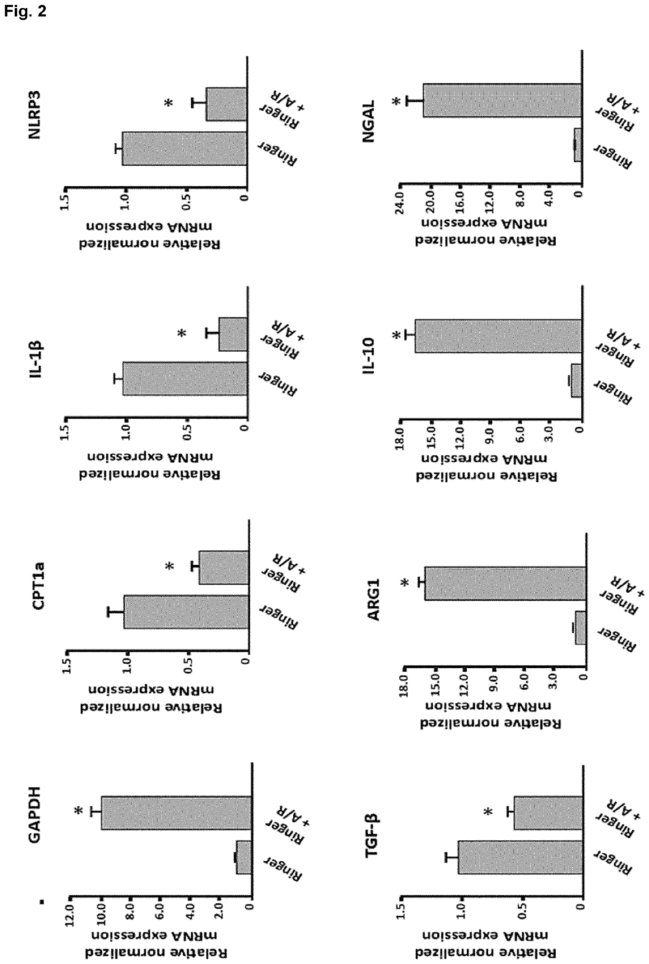

**Fig. 2****:** Gene expression profile of PBMC isolated from human blood incubated in ringer or after repeated anoxia-reoxygenation. mRNA levels of GAPDH, CPT1α, IL-1-β, NLRP3, TGF-β, Arginase1, IL-10, NGAL normalized to actine and expressed as fold change. Data were presented as mean ± SEM; *P < 0.05 vs ringer.

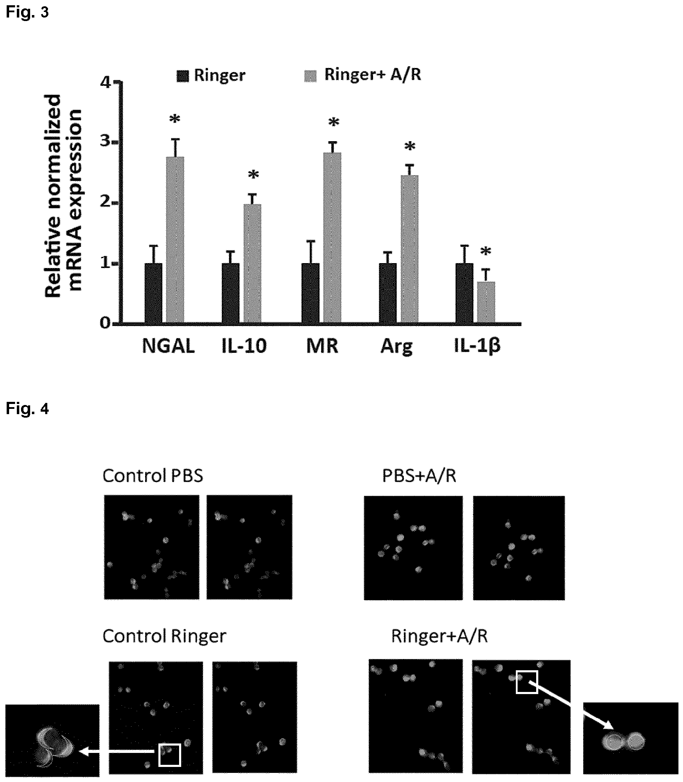

**Fig. 3****:** Gene expression profile of PBMC isolated from mice blood incubated in ringer or after repeated anoxia-reoxygenation. mRNA levels of IL-10, NGAL, Arg and Mannose receptor and IL-1β normalized to 18S and expressed as fold change. Data were presented as mean ± SEM; *P < 0.05 vs ringer.
**Fig. 4****:** Mitochondrial distribution in mouse macrophages (RAW 264.7 cell line) in different conditions. Assessed groups: medium, medium+ Anoxia reoxygenation, ringer and ringer+ Anoxia reoxygenation. Nucleous were stained with DAPI (blue) and mitochondrias with Mitotracker Green (green).

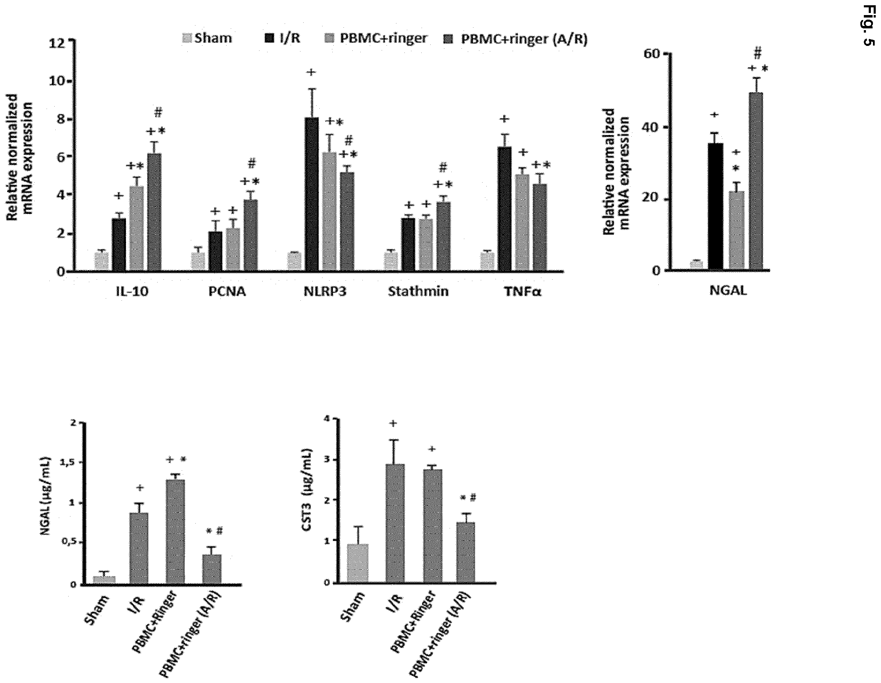

**Fig. 5****:** Animals were subjected to 30 min of bilateral ischemia or were sham-operated and sacrificed 72 h after reperfusion. PBMC infusion was administrated to their respective group 48 h after reperfusion. Inflammation and proliferation parameters were analyzed by qPCR. Renal function was assessed cystatin and NGAL in plasma on each group. Data were represented as mean ± SEM.; + P<0.05 vs Sham, * P<0.05 vs I/R, # P< 0.05 vs PBMC+ ringer.

### GENERAL DEFINITIONS

It must be noted that, as used herein, the singular forms "a", "an", and "the", include plural references unless the context clearly indicates otherwise. Further, unless otherwise indicated, the term "at least" preceding a series of elements is to be understood to refer to every element in the series. Those skilled in the art will recognize or be able to ascertain using no more than routine experimentation, many equivalents to the specific embodiments of the invention described herein. Such equivalents are intended to be encompassed by the present invention.

As used herein, the term "about" (or "around") means the indicated value ± 1 % of its value, or the term "about" means the indicated value ± 2 % of its value, or the term "about" means the indicated value ± 5 % of its value, the term "about" means the indicated value ± 10 % of its value, or the term "about" means the indicated value ± 20 % of its value, or the term "about" means the indicated value ± 30 % of its value; preferably the term "about" means exactly the indicated value (± 0 %).

As used herein, the conjunctive term "and/or" between multiple recited elements is understood as encompassing both individual and combined options. For instance, where two elements are conjoined by "and/or", a first option refers to the applicability of the first element without the second. A second option refers to the applicability of the second element without the first. A third option refers to the applicability of the first and second elements together. Any one of these options is understood to fall within the meaning, and therefore satisfy the requirement of the term "and/or" as used herein. Concurrent applicability of more than one of the options is also understood to fall within the meaning, and therefore satisfy the requirement of the term "and/or."

Throughout this specification and the claims which follow, unless the context requires otherwise, the word "comprise", and variations such as "comprises" and "comprising", will be understood to imply the inclusion of a stated integer or step or group of integers or steps but not the exclusion of any other integer or step or group of integer or step. When used herein the term "comprising" can be substituted with the term "containing" or "including" or sometimes when used herein with the term "having". Any of the aforementioned terms (comprising, containing, including, having), whenever used herein in the context of an aspect or embodiment of the present invention may be substituted with the term "consisting of", though less preferred.

When used herein "consisting of' excludes any element, step, or ingredient not specified in the claim element. When used herein, "consisting essentially of" does not exclude materials or steps that do not materially affect the basic and novel characteristics of the claim.

The terms "treatment" and "therapy", as used in the present application, refer to a set of hygienic, pharmacological, surgical and/or physical means used with the intent to cure and/or alleviate a disease and/or symptoms with the goal of remediating the health problem. The terms "treatment" and "therapy" include preventive and curative methods, since both are directed to the maintenance and/or reestablishment of the health of an individual or animal. Regardless of the origin of the symptoms, disease and disability, the administration of a suitable medicament to alleviate and/or cure a health problem should be interpreted as a form of treatment or therapy within the context of this application.

As used herein, the term "NGAL" has its general meaning in the art and refers to the Neutrophil Gelatinase-Associated Lipocalin. NGAL was shown to exist both as a 25-kDa monomer and a 45-kDa disulfide-linked homodimer, and it may also be covalently complexed with neutrophil gelatinase (also known as matrix metalloproteinase 9, MMP-9) via an intermolecular disulphide bridge as a 135-kDa heterodimeric form.

As used herein, the term "IL-10" has its general meaning in the art and refers to the Interleukin 10, also known as human cytokine synthesis inhibitory factor (CSIF). IL-10 is an anti-inflammatory cytokine.

As used herein, the term "INOS" has its general meaning in the art and refers to the inducible isoform of the nitric oxide synthase. This enzyme is involved in immune response, binds calmodulin at physiologically relevant concentrations, and produces NO as an immune defense.

As used herein, the term "MMP-9" has its general meaning in the art and refers to the Matrix metallopeptidase 9. This enzyme is a matrixin, a class of enzymes that belong to the zinc-metalloproteinases family involved in the degradation of the extracellular matrix.

As used herein, the term "TGF-β" has its general meaning in the art and refers to the Transforming growth factor beta. TGF-β is a multifunctional cytokine belonging to the transforming growth factor superfamily that includes three different mammalian isoforms (TGF-β 1 to 3, HGNC symbols TGFB1, TGFB2, TGFB3) and many other signaling proteins. TGF-β proteins can be produced by all white blood cell lineages.

As used herein, the term "ARG-1" has its general meaning in the art and refers to the enzyme arginase 1. Arg1 metabolizes L-arginine into urea and L-ornithine with downstream generation of proline and polyamines, critical for cell proliferation and collagen synthesis 11. Accordingly, Arg1+ macrophages promote wound healing and tissue fibrosis and dampen T cell activation by locally depleting L-arginine.

As used herein, the term "GAPDH" has its general meaning in the art and refers to Glyceraldehyde 3-phosphate dehydrogenase. GAPDH is a protein that functions as a glycolytic enzyme and a transferrin receptor, it additionally is involved in regulating mRNA stability.

As used herein, the term "IL-1β" has its general meaning in the art and refers to Interleukin-1 beta. IL-1β is an important mediator of the inflammatory response, and is involved in a variety of cellular activities, including cell proliferation, differentiation, and apoptosis.

As used herein, the term "CPT1α" has its general meaning in the art and refers to the Carnitine palmitoyltransferase I. CPT1α is a mitochondrial enzyme responsible for the formation of acyl carnitines by catalyzing the transfer of the acyl group of a long-chain fatty acyl-CoA from coenzyme A to I-carnitine.

As used herein, the term "NLRP3" has its general meaning in the art and refers to NLR family pyrin domain containing 3. NLRP3 is expressed predominantly in macrophages and as a component of the inflammasome, it detects products of damaged cells such as extracellular ATP and crystalline uric acid.

As used herein, the term "TNF-α" has its general meaning in the art and refers to tumor necrosis factor alpha. TNF-α is an inflammatory cytokine produced by macrophages/monocytes during acute inflammation and is responsible for a diverse range of signalling events within cells, leading to necrosis or apoptosis.

As used herein, the term "IL-6" has its general meaning in the art and refers to IL-6. IL-6 is an interleukin that acts as both a pro-inflammatory cytokine and an anti-inflammatory myokine.

By "v/v" or "volume per volume" is referred herein the volume percentage relative to the total volume of the solution or dispersion.

### DESCRIPTION OF THE EMBODIMENTS

The authors of the present invention have found a method that allows the production of macrophages polarized to a M2 phenotype (M2) which are useful in tissue repair. Said method comprises culturing the macrophages or their precursors in a medium with lactic acid or a lactate derivative, and subjecting the cells to a step of hypoxia-reoxygenation. The combination of both steps (lactate + hypoxia-re-oxygenation) results in a population of cells with a specific M2 phenotype that is different than those cells that are subjected to only one of the steps, as shown in Fig. 1.

Thus, in **a first aspect,** the present invention provides a method, from herein after "the method of the present invention", for generating cells polarized to a M2 phenotype (M2), wherein the method comprises a step of culturing a population mononuclear cells, preferably mononuclear phagocyte system cells (from now on referred to as MPS cells), in the presence of lactic acid or a lactate derivative, and a step of subjecting said cells to at least one period of hypoxia followed by a period of re-oxygenation.

Preferably, the method for generating cells polarized to a M2 phenotype comprises:
a) culturing mononuclear cells, preferably MPS cells, in a suitable culture medium comprising lactic acid or a lactate derivative, and
b) subsequently or simultaneously to step a), subjecting said cells to a period of hypoxia followed by a period of re-oxygenation.

This method of the invention may be also described as "a method for MPS cell polarization/differentiation to an M2 phenotype", "a method for obtaining MPS cells with an M2 phenotype", or "a method for obtaining activated MPS cells with a phenotype that induces tissue remodelling, regeneration or repair". By "mononuclear phagocyte system cells" or "MPS cells" is referred herein to a family of cells comprising bone marrow progenitors, monocytes, macrophages, and any precursor or progenitor cell with the potential to differentiate into a monocyte or a macrophage, including embryonic stem cells (ESCs), non-embryonic or adult stem cells, progenitor cells, and/or iPS cells. The term "bone marrow progenitor" includes, among others, myeloblast cells with the ability to differentiate to monocytes and macrophages. "Monocytes" are defined as mononuclear phagocytic cell produced in the bone marrow from the myeloid progenitors, that circulate in the blood and differentiate into macrophages upon entry into tissue. A "macrophage" is a type of immune cell that orchestrate a diverse array of functions including inflammation, tissue repair, and immune responses. For instance, they engulf and digest cellular debris, foreign substances, microbes, cancer cells, and anything else that does not have the types of proteins specific of healthy body cells on its surface in a process of phagocytosis. Macrophages are found in essentially all tissues, where they patrol for potential pathogens by amoeboid movement. They take various forms (with various names) throughout the body (e.g., histiocytes, Kupffer cells, alveolar macrophages, microglia, and others), but all are part of the mononuclear phagocyte system. Besides phagocytosis, they play a critical role in nonspecific defense (innate immunity) and also help initiating specific defense mechanisms (adaptive immunity) by recruiting other immune cells such as lymphocytes. For example, they are important as antigen presenters to T cells. Beyond increasing inflammation and stimulating the immune system, macrophages also play an important anti-inflammatory role and can decrease immune reactions through the release of cytokines. Macrophages can be identified, for example but without limitations, using flow cytometry or immunohistochemical staining by their specific expression of proteins such as, but without limitation, CD14, CD40, CD1 1 b, CD64, F4/80 (mice)/EMR1 (human), lysozme M, MAC-1 /MAC-3 and/or CD68.

The term "macrophage" as used herein includes, but without limitation, adipose tissue macrophages, monocytes, Kupffer cells, sinus histiocytes, alveolar macrophages (dust cells), tissue macrophages (histiocytes) leading to giant cells, Langerhans cells, microglia, Hofbauer cells, intraglomerular mesangial cells, osteoclasts, epithelioid cells, red pulp macrophages (sinusoidal lining cells), peritoneal macrophages, LysoMac and the like.

Macrophages can be phenotypically polarized by the microenvironment to mount specific functional programs. "Macrophage polarization" is the process wherein macrophage expresses different functional programs in response to microenvironmental signals. Although there are lots of functional states of macrophage polarization and they can be fully polarized and acquire specific phenotypes, polarized macrophages can be broadly classified in two main groups: classically activated macrophages (or M1) and alternatively activated macrophages (or M2). Macrophages that encourage inflammation are called "M1 macrophages", whereas those that decrease inflammation and encourage tissue repair are called "M2 macrophages". This difference is reflected in their metabolism; M1 macrophages have the unique ability to metabolize arginine to the "killer" molecule nitric oxide, whereas M2 macrophages have the unique ability to metabolize arginine to the "repair" molecule ornithine. M1 macrophages (previously referred to as "classically activated macrophages") are activated by LPS and IFN-gamma, and secrete high levels of IL- 12 and low levels of IL-10. In contrast, the M2 "repair" designation (also referred to as "alternatively activated macrophages") broadly refers to macrophages that function in constructive processes like wound healing and tissue repair, and those that turn off damaging immune system activation by producing anti-inflammatory cytokines like IL- 10. M2 is the phenotype of resident tissue macrophages, and can be further elevated by different stimuli such as IL-4, IL-13, immune complex plus toll-like receptor (TLR) or IL-1 receptor ligands, IL10 and glucocorticoids. M2 macrophages produce high levels of IL-10, TGF-beta and low levels of IL-12. Tumor-associated macrophages are mainly of the M2 phenotype, and seem to actively promote tumor growth. M2 macrophages are connected with Th2 immune response. They are important for encapsulation of parasites, but they are also responsible for the type II hypersensitivity. Antigen presentation is upregulated (MHC II, CD86). They also contribute on production of extracellular matrix components and tissue remodeling. Glucocorticoids influence the adherence, dissemination, apoptosis and phagocytosis of macrophages. The most common M2 macrophages are M2a, M2b and M2c macrophages, which have in common an overexpression of key cytokines such as IL-10 but differ in the expression of other inflammatory cytokines like IL 6, IL 12, TNF-α and TGB. In the present invention, a new type of M2 macrophages is described, characterized by a different cytokine expression pattern than previously known M2 macrophages. Said cytokine expression patter is further defined in the second aspect of the present invention.

The specific M2 phenotypes depend on the tissue and specific microenvironment where macrophages are. On one hand, macrophage polarization is very important for host defence against pathogen, but on the other hand it is essential for maintenance of homeostasis. Prolonged M1 type of macrophages is harmful for the organism and that is why tissue repair and restoration is necessary. M2 macrophages are responsible for that tissue repair, although they are also connected with chronic infectious diseases. A coordinate action of various inflammatory modulators, signaling molecules, and transcription factors is involved in regulating macrophage polarization. At cellular level, although M1 and M2 macrophage activities exist without T or B cell influence, specialized or polarized T cells (Th1 , Th2, Tregs) do play a role in macrophage polarized activation. Canonical IRF/STAT signaling is a central pathway in modulating macrophage polarization. Activation of IRF/STAT signaling pathways by IFNs and TLR signaling will skew macrophage function toward the M1 phenotype (via STAT1 ), while activation of IRF/STAT (via STAT6) signaling pathways by IL-4 and IL-13 will skew macrophage function toward the M2 phenotype. Signals initiated by IL-10, glucocorticoid hormones, apoptotic cell-released molecules, and immune complexes can also profoundly affect macrophage functional status. Macrophage polarization is also modulated by local microenvironmental conditions such as hypoxia. More importantly, M1-M2 polarization of macrophage is a highly dynamic process, and the phenotype of polarized macrophages can be reversed under physiological and pathological conditions. Although the M2 phenotype is generally associated to macrophages cells, in the context of the present invention, other cell types, such as monocytes, can also have a M2-polarized phenotype, as shown in the Examples. For example, a M2-polarized monocyte is characterized by differentiating to an anti-inflammatory a M2-polarized macrophage as soon as it arrives to the damaged or inflamed tissue. Thus, in the context of the present invention, the M2-polarized cell generated with the method of the present invention is a cell that, regardless of whether it is a monocyte, a macrophage, or any other MPs cell, has an anti-inflammatory M2 phenotype that is similar to the phenotype of a M2 resident macrophage. Most preferably, the M2-polarized cells generated according to the method of the present invention are the M2-polarized cells as defined in the second aspect of the present invention, whose expression profile of anti-inflammatory molecules and cytokines is explained herein.

In an embodiment, the cells cultured in step a) are mononuclear cells, preferably MPS cells, most preferably macrophages or macrophage precursors/progenitor cells. In an embodiment, the cell cultured in step a) is a M1 macrophage. In another embodiment, the cell used in step a) is a M2 macrophage, most preferably a M2a, M2b, or M2c macrophage. In a preferred embodiment, the cell cultured in step a) are peritoneal macrophages obtained from a donor or the patient to be treated. Preferably, the cells of step a) are human macrophages.

In another embodiment, the cell cultured in step a) is a differentiated or an undifferentiated macrophage. Preferably, the cell used in step a) is a macrophage precursor cell, most preferably a monocyte or a monocyte-derived cell. A "macrophage precursor or progenitor cell" is referred herein to a cell with the ability to differentiate to macrophages. Preferably, the macrophage precursor cell is a monocyte cell. Thus, in a preferred embodiment, the cells cultured in step a) are monocytes with the ability to differentiate into a macrophages, preferably with the ability to differentiate to M2 macrophages. In another preferred embodiment, the cells cultured in step a) are monocytes, more preferably previously isolated from the bloodstream of the individual to be treated or from a donor. Preferably, the monocytes are human monocytes.

Thus, the cells cultured in step a) of the method of the invention includes those macrophages produced by the differentiation of monocytes, macrophages of any differentiated or undifferentiated phenotype and any cell encompassed within the mononuclear phagocyte system, including monocytes. That is, the cells used in step a) of the method of the invention are any cells of the mononuclear phagocyte system, preferably macrophages or their precursors, such as monocytes. Said cells may be autologous, allogenic, or xenogenic. As used herein "autologous" is understood as referring to a cell preparation where the donor and the recipient are the same individual. As used herein "allogeneic" or "xenogenic" is understood as referring to a cell preparation where the donor and the recipient are not the same individual. Preferably, the cells used in step a) are isolated, human cells.

The cells used in step a) may be isolated from an individual, preferably from the bloodstream, before step a), by any mean known by those skilled in the art for obtaining biological samples comprising the desired cells, such as macrophages or their precursors, such as monocytes. Macrophages may be also isolated from any tissue (tissue resident macrophages). Macrophages are preferably isolated by intraperitoneal injection with thioglycolate. In an embodiment, the cells used in step a) are isolated from Peripheral Blood Mononuclear Cells (PBMC) isolated from blood samples (animal and human). Isolation of PBMC from blood samples is known in the art, and can be done for example by gradient centrifugation (ficoll) obtaining a product mainly composed of monocytes (CD14), T lymphocytes (CD3) and B lymphocytes (CD19). The percentage of each population of cells in human PBMCs is within the ranges described below:
- CD14 5-20%
- CD3 50-75%
- CD19 2-10%

In an embodiment, the cells used in step a) are an established cell line, preferably a macrophage or monocyte established cell line, that has not been directly or freshly isolated from a patient. Thus, in this case, the cells are immortalized cells, preferably human immortalized cells. Preferably, the cells cultured in step a) are THP-1 cells or PBMCs, as shown in the Examples.

In the context of the present invention, it is to be understood that any cell populations described herein is preferably a homogeneous or substantially pure population or a mixed cell population enriched in the specific cell population (monocytes, macrophages, etc.). By "homogeneous" or "substantially pure population" is referred as to at least about 75%, at least about 80%, at least about 85%, at least about 90%, at least about 95%, at least about 98%, at least about 99% or 100% of the cells in the composition are the cells defined herein (monocytes, macrophages, etc.). The cell populations may be added in a suitable media forming a cell suspension including pharmaceutically acceptable liquid media.

The MPS cells of step a) of the method of the invention, preferably macrophages or their precursors, such as monocytes, can be cultured by any mean and in the presence of any suitable culture medium and support of those known by the skilled in the art that are suitable for the *in vitro* maintenance and growth of said cells, as long as the culture medium comprises lactic acid or a lactate derivative. As used herein, a suitable culture medium or cultivation medium refers to a medium which is adapted to the growth requirements of the cells that are used in step a) of the method of the present invention. Suitable culturing medium to grow MPS cells, such as macrophages or monocytes, and to differentiate monocytes into macrophages, are known in the art, and some of them are described in the below examples.

As explained above, the suitable culture medium used in step a) comprises lactic acid or lactate or a lactate derivative or a salt thereof. The term "lactate derivative" comprises both enantiomeric forms, i.e. D-lactate as well as the L-lactate, wherein L-Lactate is preferred. Preferably, the lactate derivative is selected from the list comprising or consisting of sodium lactate, hypertonic lactate, calcium lactate, dimethylammonium, diethylammonium, or any lactate salt or derivative thereof. Preferably, the lactate derivative is sodium lactate, although lactate salts that contains cations like Calcium, potassium, dimethylammonium, diethylammonium, among others, can also be used. The term "lactic acid" accordingly also includes D-lactic and L-lactic acid and further includes polymeric or oligomeric forms of lactic acid such a polylactic acid (polylactate).

Although the skilled person is able to adjust the lactic acid or lactate derivative concentration in the media to provide a suitable culture medium to grow the cells used in step a), it is preferred that concentrations of between 15-45 mmol L-1, preferably between 20-30 mmol L-1, most preferably 28 mmol L-1 of lactate or lactate derivative, preferably sodium lactate, are used. Preferably the suitable culture media used in step a) comprises a mixture of sodium chloride, sodium lactate, potassium chloride, and calcium chloride in water. Most preferably, the suitable media is the Ringer's lactate medium, comprising:
- 130-131 mEq of sodium ion = 130 mmol L-1
- 109-111 mEq of chloride ion = 109 mmol L-1
- 28-29 mEq of lactate ion = 28 mmol L-1
- 4-5 mEq of potassium ion = 4 mmol L-1
- 2-3 mEq of calcium ion = 1.5 mmol L-1

Preferably the suitable culture media used in step a) is the lactate media used in ES2274438 (hypertonic lactate composition), US2019307712 (calcium lactate) and US2019247426 (calcium lactate), the entire disclosure of which, including all definitions, features and examples, explicitly and clearly belong to the description of the present invention. However, it should be understood that any other culturing medium which has been described in connection with the culture of macrophages or monocytes can be used as well. In a preferred embodiment, the suitable culture media used in step a) comprises RPMI medium supplemented with serum, preferably fetal bovine serum (FBS).

In an embodiment, the culture of step a) is carried out for a sufficient amount of time, preferably during 30-90 mins, preferably 60 min.

Step b) of the method of the present invention comprises recovering or using the MPS cells cultured in step a) and subjecting them to an intermittent process comprising a period of hypoxia followed by a period of re-oxygenation. As used herein, the term "hypoxia" refers to the presence of less than normal amounts of O₂ than those present in a vertebrate or in its blood. Preferably, "hypoxic conditions" or "hypoxia conditions" are those in which cells are subjected to oxygen concentrations of between 0 and 5%, more preferably between 0% and 0.6% (v/v), most preferably 0% O₂ (v/v). When the concentrate of O₂ is 0% is also referred to anoxic conditions. Preferably, the period of subjecting the cells to hypoxic conditions comprises subjecting the cells to anoxic conditions. More preferably, the hypoxia is induced in a hypoxic chamber under the following conditions: nitrogen 95%; CO₂ 5%; Oz 0%.

Preferably, the re-oxygenation period is carried out immediately after the hypoxic period. In an embodiment, the re-oxygenation period comprises increasing the percentage of O₂ so that the cells are not under hypoxic conditions anymore. Preferably, the re-oxygenation period comprises an increase of O₂ to concentrations of more than 5% (v/v), preferably of between 15-26% (v/v), more preferably 19-22% (v/v), most preferably between 20-21 % (v/v) (also called herein normoxia). Normoxia refers to standard oxygen conditions, in which cells are subjected to atmospheric air or to the oxygen levels commonly present in tissue culture flasks, i.e., about 20-21 % (v/v) oxygen (160 mmHg). Preferably, the reoxygenation period comprises subjecting the cells to 20-21%(v/v) of O₂, more preferably CO₂ 5% (v/v) plus atmospheric air.

Preferably, the period of hypoxia is carried out during 2 and 5 minutes, more preferably 3 minutes. Preferably, the period of re-oxygenation, preferably normoxia, is carried out during at least 45 seconds, preferably until no more than 1 minute.

As explained above, step b) comprises subjecting the MPS cultured cells of step a) (preferably macrophages or their precursors, such as monocytes) to a period of hypoxia followed by a period of re-oxygenation. Step b) can be performed several times, therefore subjecting the cells to several cycles or series of hypoxia-re-oxygenation, preferably hypoxia-normoxia. Preferably, step b) is carried out at least 2, 3, 4, 5, 6, 7, or 8, or more than 8 times. Preferably, step b) is carried out three or four times, so that the cells in step b) are subjected to three or four series of hypoxia-re-oxygenation, preferably hypoxia-normoxia. Most preferably the method consists of 4 series of hypoxia-reoxygenation. Preferably, step b) is carried out four times, so that the MPS cells, preferably macrophages or their precursors, such as monocytes, are subjected to four series of hypoxia-re-oxygenation, preferably hypoxia-normoxia, wherein the hypoxia is applied for between 2-5 minutes, preferably 3 minutes, and the re-oxygenation, preferably normoxia, is applied for between 45 seconds to no more than 1 min.

Although step b) can be performed simultaneously or subsequently to step a), it is preferred that the hypoxia-reoxygenation series of step b) are performed subsequently to step a). Further, the hypoxia-re-oxygenation series of step b) are preferably carried out with no period of time between them, so that the reoxygenation period of a previous series is immediately followed by the hypoxia period of the next series. Preferably, step b) is carried out immediately after step a).

The method of the present invention may further comprise a step c), comprising subjecting the MPS cells, preferably macrophages or monocytes, obtained after step b) to a further re-oxygenation step. This re-oxygenation step comprises culturing the cells with concentration of O₂ of between 15-26% (v/v), preferably 19-22% (v/v), most preferably between 20-21% (v/v), for at least 20, 30, 40, 50, 60, 70, 80, 90 or more minutes. Preferably, the re-oxygenation step c) is carried out during about 20, preferably 30, 40, 50, 60, 70, 80, also preferably 90, 100 mins in normoxia conditions (20-21% O₂ (v/v)).

The method of the invention may additionally comprise other steps such as, but without limitation, the maintenance and growth of MPS cells under standard culture conditions before step (a), preferably during at least 24h, and/or the maintenance and growth of the resultant MPS cells under standard conditions after step (b), preferably during at least 30 min.

The method described in the present invention comprises the *in vitro* or *ex vivo* exposure of MPS cells (preferably macrophages or their precursors, such as monocytes) to a culture comprising lactic acid or a lactate derivative or salt thereof and to repeated and consecutive series of hypoxia-reoxygenation. The resultant cells after step b), or optionally step c), acquire an activated M2 phenotype (also referred herein as M2-polarized cells). Thus, these resultant MPS cells are useful for their subsequent administration to the patient in the damaged tissue wherein they promote complete tissue regeneration. If the MPS cells cultured in step a) are macrophage precursors, such as monocytes or bone marrow progenitors, the resultant cells after the method of the present invention will be macrophage precursors, such as monocytes or bone marrow progenitors, respectively with a M2 phenotype or with the ability to differentiate to M2 macrophages. Thus, the method of the present invention may also include a further step comprising the differentiation of the cells resultant from step b), or optionally step c) to M2 polarized macrophages. This step may be performed *in vitro,* under suitable culturing conditions to differentiate macrophage precursors, such as monocytes or bone marrow progenitors, into macrophages; or *in vivo,* when the cells contact a damaged tissue that promotes their differentiation into M2 macrophages.

Preferred embodiments of the first aspect are:
In an embodiment, the method for generating cells polarized to a M2 phenotype (M2-polarized cells) comprises the steps of:
a) culturing MPS cells, preferably macrophages or monocytes, in a suitable culture medium comprising lactic acid or a lactate derivative, preferably sodium lactate, and
b) subsequently or simultaneously to step a), subjecting the cells to a period of hypoxia followed by a period of re-oxygenation, wherein re-oxygenation period is performed at an oxygen concentration of between 15-26% (v/v).

In an embodiment, the method for generating cells polarized to a M2 phenotype (M2-polarized cells) comprises the steps of:
a) culturing MPS cells, preferably macrophages or monocytes, in a suitable culture medium comprising lactic acid or a lactate derivative, preferably sodium lactate, and
b) subsequently or simultaneously to step a), subjecting the cells to a period of hypoxia followed by a period of re-oxygenation, wherein the period of hypoxia is carried out at an oxygen concentration of between 0 and 5%, preferably between 0% and 0.6%, most preferably 0% O₂ (v/v); and the period of re-oxygenation is carried out at an oxygen concentration of between 15-26%, preferably 20-35%, most preferably 20-21% (normoxia) (v/v).

In an embodiment, the method for generating cells polarized to a M2 phenotype (M2-polarized cells) comprises the steps of:
a) culturing MPS cells, preferably macrophages or monocytes, in a suitable culture medium comprising lactic acid or a lactate derivative, preferably sodium lactate, and
b) subsequently or simultaneously to step a), subjecting the cells to a period of hypoxia followed by a period of re-oxygenation, wherein the period of hypoxia lasts between 2 and 5 minutes and is carried out at an oxygen concentration of between 0-5%, preferably between 0%-0.6%, most preferably 0% O₂ (v/v); and the period of re-oxygenation lasts between 45 seconds and 1 minute and is carried out at an oxygen concentration of between 15-26%, preferably 20-35%, most preferably 20-21% (normoxia) (v/v).

In an embodiment, the method for generating cells polarized to a M2 phenotype (M2-polarized cells) comprises the steps of:
a) culturing MPS cells, preferably macrophages or monocytes, in a suitable culture medium comprising lactic acid or a lactate derivative, preferably sodium lactate, and
b) subsequently or simultaneously to step a), to a period of hypoxia followed by a period of re-oxygenation, wherein the period of hypoxia lasts 3 minutes and is carried out at an oxygen concentration of between 0-5%, preferably between 0%-0.6%, most preferably 0% O₂ (v/v); and the period of re-oxygenation lasts 45 seconds and is carried out at an oxygen concentration between 15-26%, preferably 20-35%, most preferably 20-21% (normoxia) (v/v).

In an embodiment, the method for generating cells polarized to a M2 phenotype (M2-polarized cells) comprises the steps of:
a) culturing MPS cells, preferably macrophages or monocytes, in a suitable culture medium comprising lactic acid or a lactate derivative, preferably sodium lactate, and
b) subsequently or simultaneously to step a), subjecting the cells to a period of hypoxia followed by a period of re-oxygenation, wherein the period of hypoxia lasts 3 minutes and is carried out at an oxygen concentration of between 0-5%, preferably between 0%-0.6%, most preferably 0% O₂ (v/v); and the period of re-oxygenation lasts 45 seconds and is carried out at an oxygen concentration between 15-26%, preferably 20-35%, most preferably 20-21% (normoxia) (v/v),
wherein the resulting M2-polarized cells, preferably M2-polarized macrophages or M2-polarized monocytes, are characterized by:
- having an increased expression, preferably a statistically significant increased expression, of any of IL-10, NGAL and/or ARG1, and/or
- having a decreased expression, preferably a statistically significant reduced expression, of any of GAPDH, CPT1-α, IL-1β, NLRP3 and/or TGF-β,
when compared to untreated cells, preferably untreated macrophages or monocytes, which have not been subjected to the method of the present invention.

In an embodiment, the method is a method for generating monocyte or macrophages polarized to a M2 phenotype, and it comprises the steps of:
a) culturing macrophages or monocytes in a suitable culture medium comprising lactic acid or a lactate derivative, preferably sodium lactate, and
b) subsequently or simultaneously to step a), subjecting the cells to a period of hypoxia followed by a period of re-oxygenation, wherein the period of hypoxia is carried out at an oxygen concentration of between 0 and 5%, preferably between 0% and 0.6%, most preferably 0% O₂ (v/v); and the period of re-oxygenation is carried out at an oxygen concentration of between 15-26%, preferably 20-35%, most preferably 20-21% (normoxia) (v/v).

In an embodiment, the method is a method for generating monocyte or macrophages polarized to a M2 phenotype, and it comprises the steps of:
a) culturing macrophages or monocytes in a suitable culture medium comprising lactic acid or a lactate derivative, preferably sodium lactate, and
b) subsequently or simultaneously to step a), subjecting the cells to a period of hypoxia followed by a period of re-oxygenation, wherein the period of hypoxia lasts 3 minutes and is carried out at an oxygen concentration of between 0-5%, preferably between 0%-0.6%, most preferably 0% O₂ (v/v); and the period of re-oxygenation lasts 45 seconds and is carried out at an oxygen concentration between 15-26%, preferably 20-35%, most preferably 20-21% (normoxia) (v/v).

In a **second aspect,** the present invention provides M2-polarized cells, preferably M2-polarized macrophages or M2-polarized monocytes. The cells of the second aspect are also generally called herein "M2 cells" or "M2-polarized cells of the present invention". Preferably, the M2-polarized cells of the second aspect are obtained by the method defined in the first aspect, or any of their embodiments. Said M2-polarized cells obtained by the method defined in the first aspect may be M2-polarized macrophages if the starting cells in step a) were macrophages, or they may be their precursors, such as M2-polarized monocytes, if the starting cells in step a) were said precursors.

The M2-polarized cells of the second aspect, preferably M2-polarized macrophages or M2-polarized monocytes, are characterized by an increased expression, preferably a statistically significant increased expression, of NGAL, IL10, MMP-9, ARG-1 and/or a reduced expression, preferably a statistically significant reduced expression, of GAPDH, INOS, CPT1-α, NLRP-3, IL-1β, TNF-α and TGF-β molecules compared to control cells, preferably compared to control cells which have not been subjected to the method of the present invention, also called herein control cells or untreated cells. Gene expression level may be measured or determined in cells by, for instance but without limitation, PCR, RT-LCR, RT-PCR, qRT-PCR or any other method for the amplification of nucleic acids, DNA microarrays made with oligonucleotides deposited by any method, DNA microarrays made with in situ synthesized oligonucleotides, in situ hybridization using specific labelled probes, electrophoresis gels, membrane transfer and hybridization with an specific probe, RMN, incubation with an specific antibody in assays such as Western blot, immunoprecipitation, protein arrays, immunofluorescence, immunohistochemistry, ELISA or any other enzymatic method, by incubation with a specific ligand, chromatography, mass spectrometry, and the like. By "statistically significant increased expression of a molecule" is referred herein as the determination by an analyst that the increase in the expression levels is not explainable by chance alone. By "statistically significant reduced expression of a molecule" is referred herein as the determination by an analyst that the reduction in the expression levels is not explainable by chance alone. Statistical hypothesis testing is the method by which the skilled person makes this determination. This test provides a p-value, which is the probability of observing results as extreme as those in the data, assuming the results are truly due to chance alone. A p-value of 0.1 or lower (preferably 0.05, 0.01, 0.001 or lower) is considered herein to be statistically significant. For example, the increase in the expression of the NGAL molecule in a cell, preferably in a M2 macrophage, is statistically significant when a statistical test is performed to compare it to the basal expression levels of a reference cell, preferably a macrophage not treated with the method of the present invention, as defined above, and wherein the resulting p-value of said statistical test is of 0.1 or lower, preferably 0.05, 0.01, 0.001 or lower.

Populations of M2-polarized cells of the present invention are also included in this aspect, which may substantially pure or pure, as defined above. In an embodiment, the M2-polarized cells, preferably M2-polarized macrophages or M2-polarized monocytes, of the second aspect are characterized by having a reduced expression, preferably a statistically significant reduced expression, of GAPDH in comparison to a control or untreated cell, preferably in comparison to:
- a macrophage or a monocyte that has been subjected to a hypoxia-reoxygenation treatment without having been cultured in the presence of lactic acid or a lactate derivative, and/or
- to a macrophage or a monocyte that has been cultured in a culture medium comprising lactic acid or a lactate derivative, but it has not been subjected to a hypoxia-reoxygenation treatment,
wherein the expression of GAPDH is preferably measured by RT-PCT.

In an embodiment, the M2-polarized cells, preferably M2-polarized macrophages or M2-polarized monocytes, of the second aspect are characterized by having a reduced expression, preferably a statistically significant reduced expression, of CPT1-α in comparison to a control or untreated cell, preferably in comparison to:
- a macrophage or a monocyte that has been subjected to a hypoxia-reoxygenation treatment without having been cultured in the presence of lactic acid or a lactate derivative, and/or
- to a macrophage or a monocyte that has been cultured in a culture medium comprising lactic acid or a lactate derivative, but it has not been subjected to a hypoxia-reoxygenation treatment,
wherein the expression of CPT1-α is preferably measured by RT-PCT.

In an embodiment, the M2-polarized cells, preferably M2-polarized macrophages or M2-polarized monocytes, of the second aspect are characterized by having a reduced expression, preferably a statistically significant reduced expression, of IL-1β in comparison to a control or untreated cell, preferably in comparison to:
- a macrophage or a monocyte that has been subjected to a hypoxia-reoxygenation treatment without having been cultured in the presence of lactic acid or a lactate derivative, and/or
- to a macrophage or a monocyte that has been cultured in a culture medium comprising lactic acid or a lactate derivative, but it has not been subjected to a hypoxia-reoxygenation treatment,
wherein the expression of IL-1β is preferably measured by RT-PCT.

In an embodiment, the M2-polarized cells, preferably M2-polarized macrophages or M2-polarized monocytes, of the second aspect are characterized by having a reduced expression, preferably a statistically significant reduced expression, of NLRP3 in comparison to a control or untreated cell, preferably in comparison to:
- a macrophage or a monocyte that has been subjected to a hypoxia-reoxygenation treatment without having been cultured in the presence of lactic acid or a lactate derivative, and/or
- to a macrophage or a monocyte that has been cultured in a culture medium comprising lactic acid or a lactate derivative, but it has not been subjected to a hypoxia-reoxygenation treatment,
wherein the expression of NLRP3 is preferably measured by RT-PCT.

In an embodiment, the M2-polarized cells, preferably M2-polarized macrophages or M2-polarized monocytes, of the second aspect are characterized by having a reduced expression, preferably a statistically significant reduced expression, of TGF-β in comparison to a control or untreated cell, preferably in comparison to:
- a macrophage or a monocyte that has been subjected to a hypoxia-reoxygenation treatment without having been cultured in the presence of lactic acid or a lactate derivative, and/or
- to a macrophage or a monocyte that has been cultured in a culture medium comprising lactic acid or a lactate derivative, but it has not been subjected to a hypoxia-reoxygenation treatment,
wherein the expression of TGF-β is preferably measured by RT-PCT.

In an embodiment, the M2-polarized cells, preferably M2-polarized macrophages or M2-polarized monocytes, of the second aspect are characterized by having a reduced expression, preferably a statistically significant reduced expression, of INOS in comparison to a control or untreated cell, preferably in comparison to:
- a macrophage or a monocyte that has been subjected to a hypoxia-reoxygenation treatment without having been cultured in the presence of lactic acid or a lactate derivative, and/or
- to a macrophage or a monocyte that has been cultured in a culture medium comprising lactic acid or a lactate derivative, but it has not been subjected to a hypoxia-reoxygenation treatment,
wherein the expression of INOS is preferably measured by RT-PCT.

In an embodiment, the M2-polarized cells, preferably M2-polarized macrophages or M2-polarized monocytes, of the second aspect, preferably the M2 macrophages or M2 monocytes, are characterized by having a reduced expression, preferably a statistically significant reduced expression, of GAPDH, CPT1-α, IL-1β, INOS, NLRP3 and/or TGF-β in comparison to a control or untreated cell, preferably in comparison to:
- a macrophage or a monocyte that has been subjected to a hypoxia-reoxygenation treatment without having been cultured in the presence of lactic acid or a lactate derivative, or
- to a macrophage or a monocyte that has been cultured in a culture medium comprising lactic acid or a lactate derivative, but it has not been subjected to a hypoxia-reoxygenation treatment,
wherein the expression of GAPDH, CPT1-α, IL-1β, INOS, NLRP3 and/or TGF-β is preferably measured by RT-PCT.

In an embodiment, the M2-polarized cells, preferably M2-polarized macrophages or M2-polarized monocytes, of the second aspect are characterized by having a reduced expression, preferably a statistically significant reduced expression, of TNF-α in comparison to a control or untreated cell, preferably in comparison to a macrophage or a monocyte that has been subjected to a hypoxia-reoxygenation treatment without having been cultured in the presence of lactic acid or a lactate derivative, wherein the expression of TNF-α is preferably measured by RT-PCT.

In an embodiment, the M2-polarized cells, preferably M2-polarized macrophages or M2-polarized monocytes, of the second aspect are characterized by having an increased expression, preferably a statistically significant increased expression, of IL-10 in comparison to a control or untreated cell, preferably in comparison to:
- a macrophage or a monocyte that has been subjected to a hypoxia-reoxygenation treatment without having been cultured in the presence of lactic acid or a lactate derivative, or
- to a macrophage or a monocyte that has been cultured in a culture medium comprising lactic acid or a lactate derivative, but it has not been subjected to a hypoxia-reoxygenation treatment,
wherein the expression of IL-10 is preferably measured by RT-PCT.

In an embodiment, the M2-polarized cells, preferably M2-polarized macrophages or M2-polarized monocytes, of the second aspect are characterized by having an increased expression, preferably a statistically significant increased expression, of NGAL in comparison to a control or untreated cell, preferably in comparison to:
- a macrophage or a monocyte that has been subjected to a hypoxia-reoxygenation treatment without having been cultured in the presence of lactic acid or a lactate derivative, and/or
- to a macrophage or a monocyte that has been cultured in a culture medium comprising lactic acid or a lactate derivative, but it has not been subjected to a hypoxia-reoxygenation treatment,
wherein the expression of NGAL is preferably measured by RT-PCT.

In an embodiment, the M2-polarized cells, preferably M2-polarized macrophages or M2-polarized monocytes, of the second aspect are characterized by having an increased expression, preferably a statistically significant increased expression, of ARG1 in comparison to a control or untreated cell, preferably in comparison to:
- a macrophage or a monocyte that has been subjected to a hypoxia-reoxygenation treatment without having been cultured in the presence of lactic acid or a lactate derivative, and/or
- to a macrophage or a monocyte that has been cultured in a culture medium comprising lactic acid or a lactate derivative, but it has not been subjected to a hypoxia-reoxygenation treatment,
wherein the expression of ARG1 is preferably measured by RT-PCT.

In an embodiment, the M2-polarized cells, preferably M2-polarized macrophages or M2-polarized monocytes, of the second aspect are characterized by having an increased expression of IL-10, NGAL and/or ARG1 in comparison to a control or untreated cell, preferably in comparison to:
- a macrophage or a monocyte that has been subjected to a hypoxia-reoxygenation treatment without having been cultured in the presence of lactic acid or a lactate derivative, and/or
- to a macrophage or a monocyte that has been cultured in a culture medium comprising lactic acid or a lactate derivative, but it has not been subjected to a hypoxia-reoxygenation treatment,
wherein the expression of IL-10, NGAL and/orARG1 is preferably measured by RT-PCT.

In an embodiment, the M2-polarized cells, preferably M2-polarized macrophages or M2-polarized monocytes, of the second aspect are characterized by having an increased expression, preferably a statistically significant increased expression, of MMP-9 in comparison to a control or untreated cell, preferably in comparison to a macrophage or a monocyte that has been subjected to a hypoxia-reoxygenation treatment without having been cultured in the presence of lactic acid or a lactate derivative, wherein the expression of MMP-9is preferably measured by RT-PCT.

In an embodiment, the M2-polarized cells, preferably M2-polarized macrophages or M2-polarized monocytes, of the second aspect are characterized by having a reduced expression, preferably a statistically significant reduced expression, of GAPDH, CPT1-α, IL-1β, NLRP3 and/or TGF-β, and by having an increased expression, preferably a statistically significant increased expression, of IL-10, NGAL and/or ARG1 in comparison to a control or untreated cell, preferably in comparison to:
- a macrophage or a monocyte that has been subjected to a hypoxia-reoxygenation treatment without having been cultured in the presence of lactic acid or a lactate derivative, and/or
- to a macrophage or a monocyte that has been cultured in a culture medium comprising lactic acid or a lactate derivative, but it has not been subjected to a hypoxia-reoxygenation treatment,
wherein the expression of GAPDH, CPT1-α, IL-1β, NLRP3, TGF-β, IL-10, NGAL and/or ARG1 is preferably measured by RT-PCT.

A **third aspect** refers to a composition, preferably a pharmaceutical composition, comprising a plurality of the M2-polarized cells of the second aspect. The composition of the third aspect is also called herein "the composition of the invention" or "the pharmaceutical composition of the invention". The pharmaceutical composition of the invention may additionally comprise a pharmaceutically acceptable vehicle, excipient, adjuvant and/or other active ingredient.

As used herein, the term "pharmaceutical composition" refers to a chemical or biological composition suitable for administration to a mammal. Examples of compositions appropriate for such therapeutic applications include preparations for parenteral, subcutaneous, transdermal, intradermal, intramuscular, intracoronarial, intramyocardial, intrapericardial, intraperitoneal, intravenous (e.g., injectable), intraparenchymal, intrathecal, or intratracheal administration, such as sterile suspensions, emulsions, and aerosols. Intratracheal administration can involve contacting or exposing lung tissue, e.g., pulmonary alveoli, to a pharmaceutical composition comprising a therapeutically effective amount of the M2-polarized cells of the invention, alone or in combination with other agents. In some cases, pharmaceutical compositions appropriate for therapeutic applications may be in admixture with one or more pharmaceutically-acceptable excipients, diluents, or carriers such as sterile water, physiological saline, glucose or the like. For example, the M2-polarized cells of the present invention can be administered to a subject as a pharmaceutical composition comprising a carrier solution.

The term "excipient" makes reference to a substance which aids the absorption of the elements of the composition of the invention, stabilises said elements, activates or helps to prepare the composition in the sense of giving it consistency. Therefore, excipients may have a bonding function for keeping the ingredients bonded together, such as for example in the case of starches, sugars or celluloses, a sweetening function, a dyeing function, a protective function for protecting the composition, such as for example to isolate it from the air and/or humidity, a filling function for filling a pill, capsule or any other form of presentation, such as for example in the case of dibasic calcium phosphate, a disintegrating function to facilitate the dissolution of the components and their absorption, not excluding any type of excipients not mentioned in this paragraph.

The "pharmaceutically acceptable vehicle", like the excipient, is a substance or combination of substances used in the composition to dilute any of the components comprised therein up to a certain volume or weight. The term "vehicle" refers to a solvent, coadjuvant, excipient or carrier with which the composition of the invention must be administered; obviously, said vehicle must be compatible with said composition and with the cells comprised in it. Pharmaceutically acceptable vehicles may be, but not limited to, solids, liquids, solvents or surfactants. Examples of vehicles may be, but not limited to, water, oils or surfactants, including those of petroleum, animal, vegetable or synthetic origin, such as for example, in the non-limiting sense, peanut oil, soybean oil, mineral oil, sesame seed oil, castor oil, polysorbates, sorbitan esters, ether sulfates, sulfates, betains, glucosides, maltosides, fatty alcohols, nonoxinoles, poloxamers, polioxiethylenes, poliethylenglycols, dextrose, glycerol, digitonin and similar. The pharmacologically acceptable vehicle is an inert substance or vehicle having an action similar to any of the elements comprised in the composition of the present invention. The function of the vehicle is to facilitate the incorporation of other elements, enable better dosing and administration or give consistency and format to the composition. When the format of the presentation is liquid, the pharmacologically acceptable vehicle is the solvent. The composition of the invention comprises the M2-polarized cells of the invention or the M2- polarized population of cells of the invention in a therapeutically effective amount or density. "Therapeutically effective amount" is understood to be the amount or density of M2- polarized cells of the invention or the M2-polarized cells population of the invention that, when administered to the patient to be treated, produces the desired effect, thereby promoting tissue repair and inflammation reduction. The therapeutically effective amount may vary depending on a variety of factors, for example, but not limited to, the type, severity and extension of the damage in the tissue, as well as age, physical condition, response or tolerance capacity to the cell therapy, etc., of the individual to whom the composition of the invention is going to be administered.

The composition of the invention and/or its formulations may be administered in a variety of ways including, but not limited to, parenteral, intraperitoneal, intravenous, intradermal, epidural, intraspinal, intrastromal, intraarticular, intrasinovial, intratecal, intralesional, intraarterial, intracardiac, intramuscular, intranasal, intracranial, cutaneous or subcutaneous, intraorbital, intracapsular, topic, ophthalmological or ocular, by means of surgical implant, internal surgical paint, infusion pump or via catheter.

The composition of the present invention can be formulated for administration to an animal, preferably a mammal, including humans, in a variety of ways known in the state of the art. Examples of preparations could include any solid composition (tablets, pills, capsules, bags, bottles, powders, granules, bars, pencils, vaporisers, aerosols, etc.), semi-solid (ointment, cream, balm, gel, hydrogel, foam, lotion, soap, gelatin, etc.) or liquid (aqueous or non-aqueous solutions, hydroalcoholic or hydroglycolic solutions, suspensions, emulsions, syrups, anhydrous compositions, aqueous dispersions, oils, milks, balsams, liniments, serums, etc.) for topic or parental administration, preferably parenteral administration. The composition of the present invention may also be in the form of sustained release formulations or any other conventional release system. The term "sustained release" is used in the conventional sense in reference to a compound or cell vehiculisation system that enables the gradual release of said cells during a period of time and preferably, although not necessarily, with relatively constant cell release over a period of time. Illustrative examples of sustained release vehicles or systems include, but are not limited to, liposomes, mixed liposomes, oleosomes, niosomes, etosomes, milicapsules, microcapsules, nanocapsules, sponges, cyclodextrins, blisters, micelles, mixed surfactant micelles, mixed surfactant phospholipid micelles, milispheres, microspheres, nanospheres, lipospheres, microemulsions, nanoemulsions, miniparticles, miliparticles, microparticles, nanoparticles, solid lipid nanoparticles, nanostructured lipid media, polymer materials, biodegradable or non-biodegradable patches or implants, or biodegradable microparticles, such as for example biodegradable microspheres.

The composition of the present invention is also suitable for being applied by means of medical devices which make it possible to release the M2-polarized cells of the invention or the M2-polarized population of cells of the invention in the desired area in adequate concentrations for tissue regeneration and/or reduction of tissue inflammation. These devices must be, preferably, appropriate for locally administering the cells, allowing the treatment to act on the affected region and not be dispersed. The devices can, for example, but not limited to, include the cells their interior or be coated therewith.

The M2-polarized cells of the second aspect produced with the method of the first aspect are capable of promoting tissue regeneration, inflammation reduction and scar or fibrosis clearance (fibrosis reduction). Therefore, they may be used in cell therapy methods for tissue regeneration through their administration to the injured area of the subject in need thereof. Hence, a **fourth aspect** of the present invention relates to the use of the M2-polarized cells of the second aspect, or the composition of the third aspect, or any of their embodiments, in therapy or for the manufacture of a medicament.

The use according to the fourth aspect is preferably in cell therapy, more preferably in tissue repair, tissue regeneration, tissue remodelling, wound healing, resolution of inflammation, treatment and/or repair of wounds, treatment of (tissue) inflammation, treatment of damaged, injured or impaired tissue and the like. As used herein "cell therapy" (also called "cellular therapy" or "cytotherapy") refers to the therapy in which cellular material or cells is injected into a patient; in the context of this invention this means intact, living cells. Preferably, the use according to the fourth aspect involves the use of the M2-polarized cells of the second aspect, or the composition of the third aspect, in the treatment of conditions associated with inflammation in tissue and/or organs. Preferably, said conditions associated with inflammation in tissue and/or organs are acute or chronic conditions.

Preferably, the conditions associated with inflammation or fibrosis in tissue and/or organs is selected from the list consisting of Pulmonary fibrosis, renal fibrosis, liver fibrosis, Acute respiratory distress syndrome (ARDS), ischemia/reperfusion, acute kidney injury, acute kidney injury from surgery with extracorporeal circulation, lupus, inflammatory or autoimmune diseases, among others.

Preferably, the use or treatment involves treating a tissue and/or an organ that is an inflamed or fibrotic tissue and/or organ, preferably selected from the list consisting of kidney, liver, brain, lung, skeletal muscle, skin, nervous tissue, or any combination thereof. More preferably, the tissue to be treated or repaired is skeletal muscle, even more preferable skeletal muscle injured by overuse or trauma.

Preferably, these M2-polarized cells, preferably M2 macrophages or M2 monocytes, may be injected, more preferably via intramuscular or intravenous, in the damaged area for tissue regeneration, even more preferably for muscle repair. For instance, once the resultant M2 cells, preferably M2 macrophages or M2 monocytes, are recovered in the step b) of the method of the invention, these may be administered to the individual, preferably in the damaged area, for tissue repair or they may be added to medical or pharmaceutical compositions for their use as a medicament in cell therapy in the regeneration of damaged or injured tissues.

As used herein, the terms "subject" or "patient" are used interchangeably and can encompass any vertebrate including, without limitation, humans, mammals, reptiles, amphibians, and fish. However, advantageously, the subject or patient is a mammal such as a human, or a mammal such as a domesticated mammal, e.g., dog, cat, horse, and the like, or livestock, e.g., cow, sheep, pig, and the like. Preferably, the subject is a human. As used herein, the phrase "in need thereof" indicates the state of the subject, wherein therapeutic or preventative measures are desirable. Such a state can include, but is not limited to, subjects having a disease or injury as described herein or a pathological symptom or feature associated with a disease or injury as described herein. In some cases, a method of treating or preventing a disease or injury as described herein comprises administering a pharmaceutical composition comprising a therapeutically effective amount of the macrophages of the second aspect as a therapeutic agent (i.e., for therapeutic applications).

In a **fifth aspect,** the present invention provides a method of treating an inflamed nor fibrotic tissue and/or organ, or a condition associated to inflammation in tissue and/or organs, comprising administering the M2-polarized cells, preferably M2 macrophages or M2 monocytes, as defined in the second aspect, or the composition of the third aspect, or any of their embodiments. Said method of treatment involves tissue repair, tissue regeneration, tissue remodeling, wound healing, resolution of inflammation, treatment and/or repair of wounds, treatment of (tissue) inflammation, treatment of damaged, injured or impaired tissue and the like, in a subject in need thereof that comprises administering to the subject a therapeutically effective amount of the M2 macrophages of the invention or the composition of the invention.

In a **sixth aspect,** the present invention provides a kit, hereinafter "kit of the invention", comprising the M2-polarized cells, preferably M2 macrophages or M2 monocytes, the M2-polarized cell population of the invention or the pharmaceutical composition of the invention, preferably in a therapeutically effective amount, and a medical instrument suitable for the injection of the macrophages in a tissue. A "medical instrument suitable for the injection of the macrophages in a tissue" is any medical device or instrument that may be used for the inclusion (preferably injection) of cells in a body or tissue. Examples of these devices or instruments are, but without limitations, syringes, vials, catheters, needles, cannulas, or in general any instrument used in cell therapies of those known in the art.

The M2-polarized cells, preferably M2 macrophages or M2 monocytes, of the invention, the M2-polarized cells population of the invention or the pharmaceutical composition of the invention may be encapsulated, for instance in vials, labelled and/or immobilized in a support in the kit of the invention. Additionally, the kit of the invention may comprise other elements useful for the in vitro or ex vivo maintenance of the M2-polarized cells of the invention, the M2-polarized cells population of the invention or the pharmaceutical composition of the invention in the kit.

The kit of the invention may also comprise elements that prevent the contamination of the M2-polarized cells included in it, such as antibiotic, bacteriostatic, bactericidal and/or fungicidal compounds, and the like.

The kit of the invention may also comprise other compounds, pharmaceutical compositions or medicaments suitable for the treatment of tissue damage, for tissue repair, tissue regeneration, tissue remodelling, wound healing, resolution of inflammation, treatment and/or repair of wounds, treatment of (tissue) inflammation, treatment of damaged, injured or impaired tissue and the like. These additional compounds would act as adjuvants (in combination) in the cell therapy with the M2-polarized cells, preferably M2 macrophages or M2 monocytes, of the invention, the M2-polarized cells population of the invention or the pharmaceutical composition of the invention.

Each embodiment disclosed herein is contemplated as being applicable to each of the other disclosed embodiments. Thus, all combinations of the various elements described herein are within the scope of the invention. It should also be understood that, unless clearly indicated to the contrary, in any methods claimed herein that include more than one step or act, the order of the steps or acts of the method is not necessarily limited to the order in which the steps or acts of the method are recited.

The invention is described below by the following examples, which must be considered as merely illustrative and in no case limiting of the scope of the present invention.

### EXAMPLES

### Previous considerations

All polarization experiments have been performed using either pure commercial macrophages/monocytes, or on Peripheral Blood Mononuclear Cells (PBMC) isolated from blood samples (animal and human). Isolation of PBMC from blood samples was performed by gradient centrifugation (ficoll) obtaining a product mainly composed of monocytes (CD14), T lymphocytes (CD3) and B lymphocytes (CD19). The percentage of each population of cells is inside the ranges described below:
- CD14 5-20%
- CD3 50-75%
- CD192-10%

The Lactate used in all the examples is ringer lactate, the most common medium enriched in lactate used in clinical practice, and whose composition is described below:
- 130-131 mEq of sodium ion = 130 mmol L-1
- 109-111 mEq of chloride ion = 109 mmol L-1
- 28-29 mEq of lactate ion = 28 mmol L-1
- 4-5 mEq of potassium ion = 4 mmol L-1
- 2-3 mEq of calcium ion = 1.5 mmol L-1

Other lactate solutions commercially used, are peritoneal dialysis solutions like Dianeal, Physioneal and Nutrineal. Furthermore, several patents have also described the use of different types of lactate solutions like ES2274438 (hypertonic lactate composition), US2019307712 (calcium lactate) and US2019247426 (calcium lactate)

### 1. Comparative experiments

In order to study the effect of ringer lactate and anoxia re-oxygenation + ringer lactate on macrophage/monocyte polarization, the following experiments were performed using different types of macrophages or mononuclear cells (commercial monocytes, and mononuclear cells for human and mice blood). Several RT-PCRs were run in order to assess the CPT1- α, IL-1β, NLRP3, TNF- α, NGAL, IL10, iNOS, MMP-9, TGF-β and ARG1 expression in each group.

### 1.1. Gene expression profile of human monocytes (THP-1 cell line) in different conditions.

### 4 different groups were tested:

1. Human Monocytes in Medium under oxygen standard conditions (CO2 5% plus atmospheric air). (Medium)
2. Human Monocytes in medium subjected to 4 anoxia (nitrogen 95%; CO2 5%; O2 0%).-re-oxygenation series plus 1h and 30min of re-oxygenation. (Medium + A/R)
3. Human Monocytes in ringer lactate under oxygen standard conditions (CO2 5% plus atmospheric air). (Ringer)
4. Human Monocytes in ringer lactate subjected to 4 anoxia (nitrogen 95%; CO2 5%; O2 0%) -re-oxygenation series plus 1h and 30min of re-oxygenation. (Ringer + A/R)

The different expression of markers is described in figure 1. Significant differences were observed between ringer and ringer + A/R.

### 1.2. Gene expression of Peripheral Blood mononuclear cells (PBMC) isolated from human blood.

### 2 different groups were tested:

1. Human PBMC in ringer lactate under oxygen standard conditions (CO2 5% plus atmospheric air). (Ringer)
2. Human PBMC in ringer lactate subjected to 4 anoxia (nitrogen 95%; CO2 5%; O2 0%).-re-oxygenation series plus 1h and 30 min of re-oxygenation. (Ringer + A/R)

The different expression of markers is described in **figure 2****.** Significant differences were observed between ringer and ringer + A/R in all the measured genes.

### 1.3. Gene expression profile of PBMC isolated from mice blood

### 2 different groups were tested:

1. mice PBMC in ringer lactate under oxygen standard conditions (CO2 5% plus atmospheric air). (Ringer)
2. mice PBMC in ringer lactate subjected to 4 anoxia (nitrogen 95%; CO2 5%; O2 0%).-re-oxygenation series plus 1h and 30min of re-oxygenation. (Ringer + A/R)

The different expression of markers is described in **figure 3****.** Significant differences were observed between ringer and ringer + A/R in all the measured genes.

### 1.4. Mitochondria distribution in mouse macrophages (RAW 264.7 cell line)

According to **figure 4****,** mitochondrial distribution was also analyzed using Mitotracker Green dye in 4 different groups:
1. Mouse macrophages in Medium under oxygen standard conditions (CO2 5% plus atmospheric air). (Medium)
2. Mouse macrophages in medium subjected to 4 anoxia (nitrogen 95%; CO2 5%; O2 0%).-re-oxygenation series plus 1h and 30min of re-oxygenation. (Medium + A/R)
3. Mouse macrophages in ringer lactate under oxygen standard conditions (CO2 5% plus atmospheric air). (Ringer)
4. mouse macrophages in ringer lactate subjected to 4 anoxia (nitrogen 95%; CO2 5%; O2 0%).-re-oxygenation series plus 1h and 30min of re-oxygenation. (Ringer + A/R)

### 2. Effect of PBMC in ringer lactate and submitted to anoxia deoxygenation on animal models of ischemia reperfusion injury.

Animals were subjected to 30 min of bilateral ischemia, or were sham-operated and sacrificed 72 h after reperfusion. PBMC were obtained as described above. PBMC infusion was administrated to their respective group 48 h after reperfusion. Inflammation (IL 10, TNF- α, NLRP3) and proliferation (NGAL in tissue, Stathmin and PCN) parameters were analyzed by qPCR. Renal function was assessed measuring cystatin and NGAL in plasma on each group.

The results, as described in **figure 5****,** show a decrease of pro-inflammatory markers (TNF-α, NLRP3), an increase of anti-inflammatory markers (IL-10) and an increase of regenerative markers (stathmin and, NGAL in tissue and PCN). Renal function markers levels were also recovered after M2 PBMC administration.

## Claims

1. A method for generating cells polarized to a M2 phenotype, the method comprising the steps of:
a) culturing mononuclear phagocyte system cells in a suitable culture medium comprising lactic acid or a lactate derivative, and
b) subsequently or simultaneously to step a), subjecting the cells to a period of hypoxia followed by a period of re-oxygenation.

2. The method according to claim 1, wherein the mononuclear phagocyte system cells are macrophages or macrophage precursors, preferably monocytes.

3. The method according to any one of claims 1 or 2, wherein step b) is performed at least four times.

4. The method according to any one of claims 1 to 3, wherein the re-oxygenation period comprises applying normoxia conditions (20-21% (v/v) oxygen) to the cells.

5. The method according to any one of claims 1 to 4, wherein the hypoxia period is carried out at an oxygen concentration of between 0 and 5 % (v/v).

6. The method according to any one of claims 1 to 5, wherein the hypoxia period lasts between 2 and 5 minutes.

7. The method according to any one of claims 1 to 6, wherein the re-oxygenation period lasts between 45 seconds and 1 minute.

8. The method according to any one of claims 1 to 7, wherein the suitable culture medium of step a) comprises sodium lactate.

9. A population of cells polarized to a M2 phenotype obtained or obtainable from the method defined in claims 1 to 8.

10. The population of cells according to claim 9, **characterized by** having an increased expression of any of NGAL, IL-10, MMP-9 and/or ARG-1 molecules, or any combination thereof, when compared to a control or untreated cell, preferably when compared to a control cell that has not been subjected to the method defined in any one of claims 1 to 8.

11. The population of cells according to any one of claims 9 or 10, **characterized by** having a reduced expression of any of GAPDH, CPT1-a, NLRP-3, IL-1β, INOS and/or TGF-β molecules, or any combination thereof, when compared to a control or untreated cell, preferably when compared to a control cell that has not been subjected to the method defined in any one of claims 1 to 8.

12. A pharmaceutical composition comprising the population of cells defined in any one of claims 9 to 11.

13. A pharmaceutical composition as defined in claim 12, for use in medicine.

14. A pharmaceutical composition as defined in claim 12, for use in the treatment of conditions associated with inflammation in tissue and/or organs.

15. The pharmaceutical composition for use according to any of claims 13 to 14, wherein the conditions associated with inflammation in tissue and/or organs is selected from the list consisting of pulmonary fibrosis, renal fibrosis, liver fibrosis, acute respiratory distress syndrome (ARDS), ischemia/reperfusion, acute kidney injury, acute kidney injury from surgery with extracorporeal circulation, lupus, inflammatory or autoimmune diseases.
